# EUROPEAN PATENT SPECIFICATION

(11) **EP 4 181 994 B1**
(45) Date of publication and mention of the grant of the patent: **20.08.2025**
(21) Application number: 21745989.0
(22) Date of filing: 15.07.2021
(51) Int. Cl.: A61M 16/04, A61B 17/34, A61M 29/02

(54) **KIT OF COMPONENTS FOR EXTRUSIVE PERCUTANEOUS DILATIONAL TRACHEOTOMY**
KOMPONENTENKIT FÜR DIE EXTRUSIVE PERKUTANE DILATATIONSTRACHEOTOMIE
KIT DE COMPOSANTS POUR TRACHÉOTOMIE PERCUTANÉE PAR DILATATION EXTRUSIVE

(30) Priority: 17.07.2020 IT 202000017461
(43) Date of publication of application: 24.05.2023
(73) Proprietor: Maggio, Giuseppe, 27050 Corvino San Quirico (PV) (IT)
(72) Inventor: Maggio, Giuseppe, 27050 Corvino San Quirico (PV) (IT)
(74) Representative: Botti & Ferrari S.p.A.
(86) International application number: PCT/EP2021/069885
(87) International publication number: WO 2022/013403

(56) References cited:
- US-A- 5 078 743
- US-A1- 2016 220 774
- US-B1- 9 555 206

## Description

### Field of application

In its most general aspect, the present invention relates to the field of intensive medicine.

In particular, the present invention relates to a kit for percutaneous dilational tracheotomy that allows performing an extrusive tracheotomy.

### Prior art

During the hospitalization of patients in intensive care it is often necessary to perform a tracheotomy to manage respiratory failure, to facilitate weaning from mechanical ventilation or to protect the airways.

Tracheotomy is a surgical procedure that allows opening an access to the trachea (stoma) and that can be performed with open surgery or with percutaneous dilational techniques.

Compared to the surgical technique, percutaneous dilational techniques are faster and accessible even to specialists other than the ENT surgeon.

The techniques for performing a percutaneous dilational tracheotomy are divided into two groups, intrusive tracheotomies and extrusive tracheotomies.

In both cases (both for intrusive and extrusive) percutaneous dilational tracheotomies begin with the insertion of a large needle from the outside into the central part of the neck, until the tip is introduced into the lumen of the trachea through its front wall, in a central point and perfectly localized between two of the first tracheal rings. This maneuver is delicate and crucial for the success of the whole operation. For this reason, it is performed under tracheoscopic guidance (that is, directly viewing the trachea from the inside) with a fibrobronchoscope or rigid optics.

After the puncture of the trachea, a guide wire is inserted into the large needle according to the Seldinger technique, which must be pushed in one or the other direction depending on the type of percutaneous dilational tracheotomy to be performed: towards the keel (i.e. towards the distal part of the trachea) if an intrusive tracheotomy is being performed, towards the cranial part of the patient to come out of the mouth if instead an extrusive tracheotomy is being performed.

In intrusive tracheotomies, the tracheal and pretracheal tissues are progressively dilated from the outside towards the inside of the trachea. Conversely, in extrusive tracheotomies the tracheal and pretracheal tissues are progressively dilated from the inside of the trachea towards the outside.

Intrusive tracheotomies are easier and faster to perform and learn, for these reasons they are nowadays the most used.

However, intrusive tracheotomies expose to the risk of rupture of the tracheal rings and the rear wall of the trachea due to the direction of access to the trachea, that is from the outside towards the inside. In addition, intrusive tracheotomies expose to a greater risk of bleeding and infection of the stoma since they require large skin incisions and greater dilation of the tracheal and pretracheal tissues.

On the contrary, extrusive tracheotomies, acting from the inside of the trachea towards the outside, reduce the risk of rupture of the tracheal rings and the rear wall of the trachea; moreover, extrusive tracheotomies require minimal or even no incisions, thereby limiting the risk of bleeding and infection of the stoma.

These advantages of extrusive tracheotomies are indispensable especially in those clinical contexts, increasingly frequent in modern intensive care, with increasingly elderly and complex patients, often treated with anticoagulant and antiplatelet drugs.

A currently known extrusive tracheotomy technique called translaryngeal tracheotomy (TLT) was disclosed in the document: "Intensive Care Med 23: 386-392 © Springer-Verlag 1997 "A non-derivative, non-surgical tracheostomy: the translaryngeal method" A. Fantoni, D. Ripamonti".

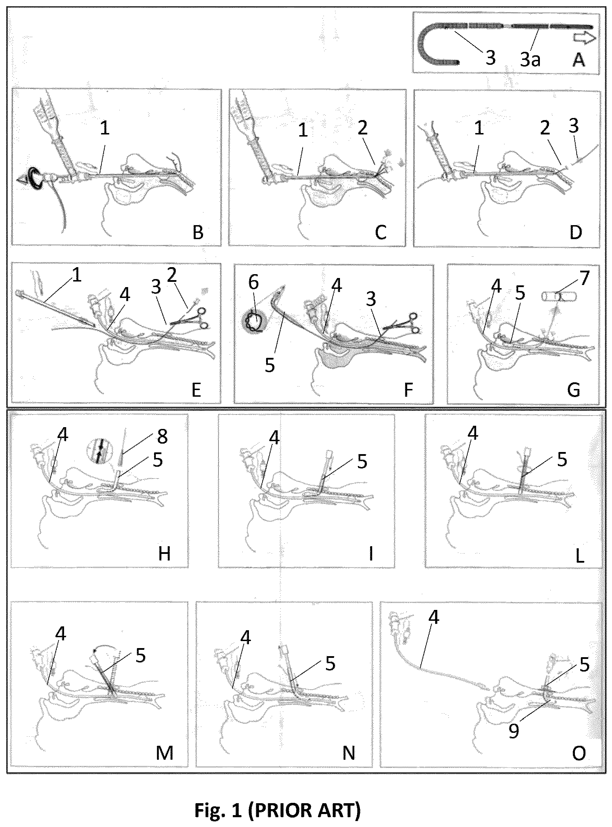

The steps for performing this TLT extrusive technique, described below with reference to Figure 1 (A-O), include:
1. insertion from the mouth of a rigid tracheoscope 1 to allow the use of rigid optics and in order to stabilize and protect the trachea (Fig. 1B) or alternatively use of a fibrobronchoscope and the tube already present in the patient's airways (not shown);
2. puncture of the trachea with a needle 2 under direct tracheoscopic vision (Fig. 1C);
3. insertion of a guide wire 3 into the needle 2 (Fig. 1D);
4. extraction of the guide wire 3 from the mouth with cutting of its proximal part and reintubation with a ventilation small tube 4 (Fig. 1E);
5. insertion and locking of the guide wire 3 inside a dilating cone-cannula 5 by means of a knot 6 (Fig. 1F);
6. creation of the stoma by advancing the cone-cannula 5, anchored to the steel guide wire 3 and tied to the knob 7, from the inside to the outside of the trachea with a controlled maneuver (Fig. 1G);
7. cutting of the pointed end 8 of the cone-cannula 5 with which the stoma was created (Fig. 1H);
8. partial extraction of the cannula 5 from the airways (Fig. 1I);
9. rotation of the cannula 5 (Fig. 1L);
10. overturning of the cannula 5 (Fig. 1M);
11. reinsertion of the cannula 5 into the airways to orient it in the correct position towards the bronchi (Fig. 1N);
12. connection of the small tube for inflating a cuff 9 of the cannula 5 (Fig. 1O);
13. removal of the ventilation small tube 4 and final ventilation through the new cannula 5 positioned in place (Fig. 1O).

The TLT extrusive tracheotomy technique just described, despite the above-mentioned advantages, is currently out of use due to some technical difficulties in its execution that make it difficult to learn, more insidious and time-consuming to perform compared to the intrusive tracheotomy techniques currently in use.

In detail, the disadvantages of TLT extrusive tracheotomy compared to intrusive tracheotomies are:
- longer duration: 30-40 minutes compared to 15-20 minutes;
- need for double reintubation (Fig. 1B and 1E): higher risk of complications and inapplicability in emergency situations;
- complex management of the guide wire 3: it is welded to a braided steel cable 3a (Fig. 1A) from which it is separated (Fig. 1E) to allow the insertion of the braided steel cable 3a into the cone-cannula 5 (Fig. 1F) and its subsequent knotting (Fig. 1F) and anchoring to the knob (1G);
- cutting of the cone-cannula 5 (Fig. 1H): risk of cutting the guide wire 3 and the small tube of the cuff necessary to inflate the cuff 9 of the cannula 5;
- reorientation maneuver of the cannula 5 (Fig. 1I-1N): risk of accidental extraction of the cannula 5 with immediate closure of the stoma;
- cannula 5 provided with a metal spiral: incompatibility with RMN, impossibility of replacement with another type of cannula during the execution of the tracheotomy, possibility of replacement with another cannula ideally only after 5-7 days from the tracheotomy.

Most of the technical difficulties related to the TLT extrusion technique are due to problems linked to the maneuverability of the above-described components of the TLT extrusion technique kit during the execution of the tracheotomy.

The document US 2016/0220744 A1 discloses a method and a kit relating to an endotracheal tube with a tip suitable for traversing the laryngeal inlet and with a guiding channel having a proximal opening adjacent to a proximal end portion and a distal opening adjacent to a tip, wherein the guiding channel is adjacent to a sidewall of the main lumen of the tube.

The main object of the present disclosure is thus to provide a kit of components for extrusive percutaneous dilational tracheotomy which allows performing an extrusive tracheotomy in a simpler way, with maneuvers that are more similar to those commonly used in intrusive tracheotomy techniques, and at the same time in an accurate and safe manner, so as to overcome the aforementioned drawbacks.

Another object of the present disclosure is to provide a kit of components for extrusive percutaneous dilational tracheotomy which includes interchangeable components with kit components already in use in the field of intensive medicine.

A further object of the present disclosure is to provide a kit of components for extrusive percutaneous dilational tracheotomy that it is simple and economical to make.

### Summary of the invention

The invention is defined by the appended claims.

These objects are achieved through a kit for extrusive percutaneous dilational tracheotomy comprising a dilator tube extended along a longitudinal axis and having a dilation tapered portion ending with a tip and a guide tubular portion, characterized in that said dilation tapered portion and said guide tubular portion are separatable from each other along at least one weakening line transversely extended with respect to said longitudinal axis, the guide tubular portion further comprising, in proximity to said weakening line, hooking means for a mandrel associated with a cannula.

In an embodiment of the kit of components according to the technology, the tapered portion of the dilator tube has a channel therein that is longitudinally extended from the tip and unidirectional locking means for a guide wire in said channel.

In an embodiment of the kit of components according to the technology, the at least one weakening line between the dilation tapered portion and the guide tubular portion of said dilator tube is transversely obliquely extended so as to form, after separation, a guide portion (or guide tube) with a beveled end having a substantially flute-beak-like cross section and having said hooking means in proximity to said beveled end, the kit further comprising a cannula having a beveled end having a profile that is complementary to that of the bevelled end of said guide tubular portion.

In an alternative embodiment, the at least one weakening line between the dilation tapered portion and the guide tubular portion of said dilator tube is perpendicularly extended with respect to the longitudinal axis so as to form, after separation, a guide portion (or guide tube) with a non-beveled end and having said hooking means in proximity to said non-beveled end, and the guide tubular portion further having a longitudinal notch in proximity to said weakening line, said longitudinal notch being substantially opposite said hooking means. In said embodiment, the kit of components may further comprise a conventional cannula with a non-beveled end and i.e. having a substantially right-angled cross section.

In another embodiment, the at least one weakening line is made of a weakening line that is transversely obliquely extended with respect to the longitudinal axis of the dilator tube and of a weakening line that is perpendicularly extended with respect to the longitudinal axis of the dilator tube.

Preferably, the at least one weakening line is at least one incision line.

Advantageously, this allows choosing the most suitable weakening line (or incision line) for the separation of the guide tubular portion based on the cannula that the doctor wishes to use to perform the tracheotomy. In case of use of the transversely oblique incision line to obtain a guide portion with a flute-beak-like beveled portion, the doctor can choose a cannula with a flute-beak-like beveled portion having a complementary profile, cannula which is advantageously included in the kit according to the technology. Vice versa, in case of use of the perpendicular incision line to obtain a guide portion with a non-beveled end, the doctor can choose any cannula with a non-beveled end in common use, which is not necessarily supplied with the kit according to the technology.

Preferably, the cannula has a curved tubular body ending with a flute-beak-like beveled or non-beveled distal end and an opposite proximal end, a flange in proximity to the proximal end and a cuff in proximity to the distal end in fluid communication with a balloon through a small tube. Preferably, the cannula may further have a safety opening known as a "Murphy eye" in proximity to the distal end, which allows the passage of air in the event that the distal end inserted in the trachea closes by leaning against the wall of the trachea itself.

The kit according to the technology may further comprise at least one mandrel to be used in association with a corresponding cannula, the mandrel comprising a rod with a proximal end and a distal end, a maneuvering knob at the proximal end and an anchoring element provided with a cavity at the opposed distal end, the anchoring element being adapted to removably engage with the hooking means of the guide portion of the dilator tube.

Advantageously this allows a quick, easy, and firm anchoring of the cannula to the guide tube. Said cavity of the mandrel is further adapted to disanchor from the hooking means in the tube guide by tilting the cannula after its insertion into the trachea.

Advantageously this allows a quick unhooking of the tube from the cannula from the guide tube, no longer necessary when the cannula is in the trachea.

In an embodiment, the rod of the mandrel is curved and has a fixed length with curvature and length substantially corresponding to those of a curved cannula which it is intended to be associated with.

In another embodiment, the rod of the mandrel is curved or rectilinear with variable length and the mandrel comprises a system for adjusting the length of the rod.

Advantageously, this allows hooking any commercial cannula to the guide tube without having to use the cannula with flute-beak-like ends supplied in the kit.

The kit according to the technology may comprise further components to perform the extrusive dilational percutaneous tracheotomy selected among guide wire, a ventilation small tube, at least one needle and a tracheoscope.

The guide wire has a "J"-shaped soft tip, a cable, and a knob. Said "J"-shaped soft tip is separatable from the braided steel cable while, preferably, said knob is firmly connected to said cable. Said guide wire is easily locked/hooked inside the tip of the dilator tube that has a channel therein with a unidirectional locking system.

Advantageously, this simplifies handling and pulling the guide wire throughout the tracheotomy procedure.

Further features and advantages of the present technology will be evident from the following description of some embodiments given by way of non-limiting example with reference to the attached drawings.

### Brief description of the drawings

In these drawings:
- Figure 1 represents the steps (B-O) necessary to perform an extrusive dilational percutaneous tracheotomy according to the prior art;
- Figure 2 shows an embodiment of a kit for extrusive tracheotomy according to the present technology;
- Figure 3 shows the dilator tube of the kit for extrusive tracheotomy of Figure 2;
- Figure 4 shows the dilator tube of the kit for extrusive tracheotomy of Figure 2 in detached parts;
- Figure 5 shows the cannula and mandrel of the kit for extrusive tracheotomy of Figure 2;
- Figure 6 shows, for two different types of cannulas, a detail of the hooking/unhooking means of the mandrel/cannula assembly with the guide portion separated from the dilator tube of the kit for extrusive tracheotomy of Figure 2;
- Figures 7a-7c show respective alternative embodiments of the mandrel of the kit for extrusive tracheotomy according to the technology;
- Figure 8 shows the mandrel of Figure 7c during the hooking step to the guide portion separated from the dilator tube and the unhooking step from said guide portion;
- Figure 9 shows some salient steps (A-F) to perform the extrusive percutaneous dilational tracheotomy through the kit of the present technology;

For better understanding and clarity of the figures the elements represented therein may not be on a scale representative of reality. Furthermore, similar elements in the figures will be identified by similar reference numbers.

### Detailed description

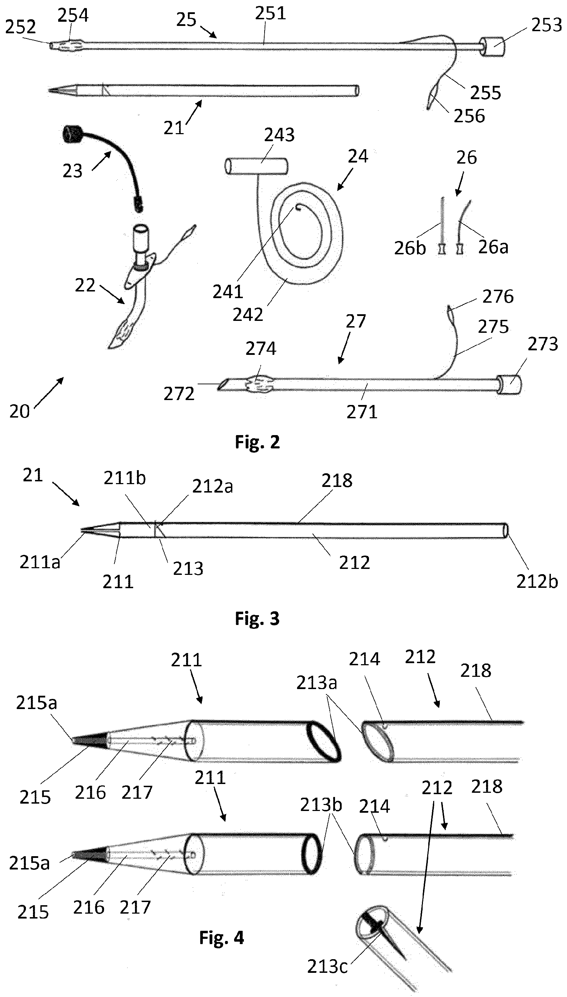

With reference to Figures 2-8, a kit of components for performing an extrusive percutaneous dilational tracheotomy according to some embodiments of the present disclosure is now herein described in detail, said kit being globally indicated with reference number 20.

The kit 20 comprises a dilator tube 21, a cannula 22, a mandrel 23, a guide wire 24, a ventilation small tube 25, at least one needle 26 and a tracheoscope 27 according to a possible embodiment of the present disclosure.

Figures 3 and 4 show an overview and a detailed view of the dilator tube 21 of the kit 20 for extrusive tracheotomy.

The dilator tube 21 has a tapered portion 211, hereinafter referred to as dilation portion 211, and a guide portion 212, hereinafter also referred to as guide tube 212, which are separatable from each other at a weakening line 213, for instance an incision line, to facilitate the separation thereof.

The tapered portion 211 has a conical section ending at a first end 211a thereof with a tip 215 having an opening 215a, and a cylindrical section connected to the above conical section ending at a second end 211b at the incision line 213. From the opening 215a of the tip 215 a channel 216 provided with unidirectional locking means 217 longitudinally departs inside the conical section of the tapered portion 211.

The opening 215a allows the entry of the guide wire 24 into the channel 216 of the tapered portion 211 of the dilator tube 21. Said guide wire 24 may slide inside the channel 216 where it encounters the locking means 217, thus remaining firmly anchored to the channel 216 of the tapered portion 211 of the dilator tube 21. As it will be seen later, the energetic traction of the guide wire 24 anchored to the channel 216 allows, during the tracheotomy procedure, the creation of the tracheal stoma.

The unidirectional locking means 217 are per se conventional and may be for instance ratchet locking, ball locking, arrow locking, arrow and wing locking, pine locking means.

Advantageously, this allows for an easy, quick, and firm anchoring between the guide wire 24 and the tapered portion 211 of the dilator tube 21, thus overcoming the cumbersome management of the guide wire 3 as in the known art, which is critical for the success of the TLT tracheotomy.

The shape of the tapered portion 211 of the dilator tube is adapted to facilitate the progression in the tracheal tissues during the dilation step of the tracheotomy, which leads to the formation of the tracheal stoma.

The dilation tapered portion 211 is preferably made of biocompatible rigid plastic and may preferably be externally coated with a hydrophilic material commonly used in the dilators of some intrusive tracheotomies, so as to make it more slippery and to favor the progression of the dilator tube 21 in the tracheal tissues.

The tip 215 of the dilation tapered portion 211 is made of metal material, preferably of stainless steel, to favor the dilation of the tracheal tissues.

The channel 216 may be made of hard plastic or a metal material.

The guide portion 212 of the dilator tube 21 has a cylindrical-shaped elongated and flexible body, without preformed curves, open at both ends 212a, 212b and has hooking/unhooking means therein of an assembly made of the mandrel 23 and the cannula 22 located in the proximity to the end 212a thereof.

In the present embodiment, said hooking/unhooking means include a tooth 214 inside the guide portion 212 in the proximity to the end 212a thereof.

Moreover, the guide portion 212 has at the outer surface thereof and on the entire length a longitudinal demarcation line 218 that helps the doctor in the correct orientation of the dilator tube 21 for the insertion into the trachea, in particular said line 218 identifies the part of the tube that must be held towards the front area of the patient's neck.

The length of the guide portion 212 is suitably sized to get out of the patient's mouth during the entire tracheotomy procedure, i.e. it will have a longer length for adult kits and a shorter length for pediatric use kits.

The diameter of the guide portion 212 is suitably sized to adapt to the different types of patient, for instance a larger diameter for adult kits and a smaller diameter for pediatric use kits and to allow the insertion of a fibrobronchoscope from the outside of the patient's mouth towards the work area at the tracheal level.

The guide portion 212 of the dilator tube 21 is preferably made of biocompatible flexible transparent plastic, preferably of PVC, silicone, or similar materials.

The incision line 213 placed between the dilation portion 211 and the guide tube 212 is useful for guiding and facilitating the separation of the dilation portion 211 from the guide portion 212, after the creation of the tracheal stoma by means of the dilation portion 211, at the ends 211b, 212a thereof. Further to the separation, the end 212a of the guide portion 212 is available to engage with the cannula 22 and the mandrel 23 associated with each other.

In an embodiment (Fig. 4), the incision line 213 present on the dilator tube 21 is transversely obliquely extended with respect to the longitudinal axis of the dilator tube 21 so as to form, after separation of the dilation portion 211 from the guide portion 212, a guide portion 212 with a beveled end 213a having a substantially flute-beak-like cross section and having the hooking/unhooking means (tooth 214) in the proximity to said beveled end 213a. In particular, in the present embodiment, the hooking/unhooking means are located internally on the longest side of the guide portion 212 at the beveled portion 213a.

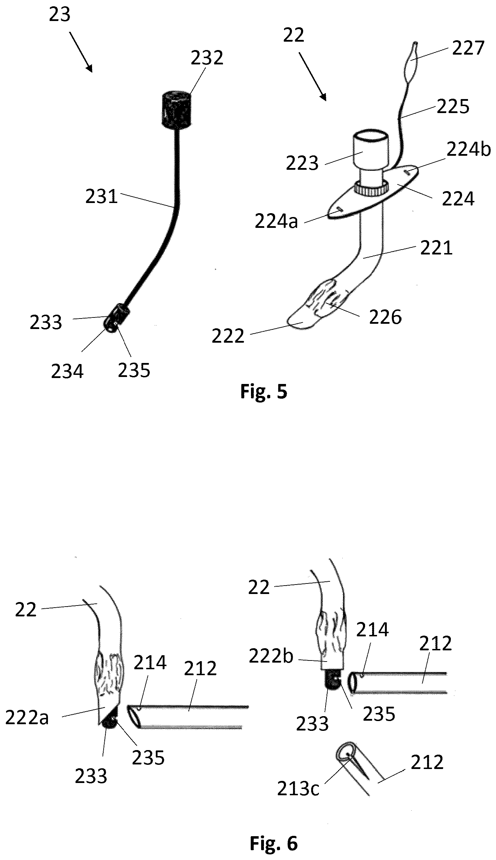

As it will be better illustrated hereinafter, said guide portion 212 is suitable for use with a cannula 22 as illustrated in Figure 5 associated with a mandrel 23 wherein the cannula 22 has a beveled end 222 having a profile that is complementary to that of the beveled end 213a of the guide portion.

In another embodiment (Fig. 4), the incision line 213 present on the dilator tube 21 is perpendicularly extended with respect to the longitudinal axis of the dilator tube 21, so as to form, after separation of the dilation portion 211 from the guide portion 212, a guide portion 212 having a non-beveled end 213b (i.e. with right-angle cross section) and bearing the hooking/unhooking means (tooth 214) in the proximity to said right-angle end 213b. In this embodiment, a notch 213c is also preferably provided on the guide portion 212 in a position substantially opposite the hooking/unhooking means (tooth 214).

As it will be better illustrated hereinafter, said guide portion 212 is suitable for use with any commercial cannula 22 commonly used in hospitals and having a non-beveled end 222b associated with a mandrel 23.

In a further embodiment, the dilator tube 21 may have both an incision line 213 obliquely extended, and an incision line 213 extended perpendicularly, as visible in Figure 3.

Advantageously, this allows the doctor to choose, by cutting the dilator tube 21 along the appropriate incision line, the preferred or most suitable cannula for tracheotomy without any constraint on the use of cannula 22 contained in the kit 20.

In this way, the kit of components 20 according to the technology may also be advantageously integrated with kits/ commercial components used in intensive medicine.

Figure 5 shows an overall view of the cannula 22 and mandrel 23 of the kit 20 for extrusive tracheotomy according to an embodiment of the present disclosure.

The cannula 22 comprises a curved tubular body 221, a first end or distal end 222, a second end or proximal end 223, a flange 224 in proximity to the second end 223 and a small tube 225.

The first end 222 of the cannula 22 has a beveled cross section, substantially in the shape of a flute beak complementary to the beveled end 213a of the guide portion 212 after the separation thereof from the dilation portion 211 along the incision line 213a.

The first end 222 of the cannula 22 is adapted to be juxtaposed to the beveled end 213a of the guide portion 212 by coupling between complementary profiles and removably engaged, thought the mandrel 23, to the guide tube 212, so that the cannula 22 and the guide tube 212 are adjacent in use.

The first end 222 of the cannula 22 may preferably have a further safety opening (not shown) known as a "Murphy eye", which allows the passage of air in the event that the end 222 inserted in the trachea closes by leaning against the wall of the trachea itself after the separation of the cannula 22 from the guide tube 212.

The second end 223 of the cannula 22 is provided with a universal fitting for the mechanical ventilation since it is intended to remain external to the trachea to engage with automatic or manual ventilation tools.

The curved tubular body 221 has at the first end 222 a cuff 226 in fluid communication with the small tube 225. The small tube 225 runs in the thickness of the wall of the curved tubular body 221 of the cannula 22 ending with a balloon 227.

The cuff 226 is therefore inflatable through the balloon 227 and, once inflated, it generates a tight coupling with the trachea, thus allowing the use of positive pressure ventilation.

The flange 224 is usually an oval-shaped soft foil and has two opposite holes 224a and 224b. The flange 224 is movable and perpendicular with respect to the tubular body 221 of the cannula 22 and is adjustable to be adapted to any type of patient. The flange 224 is the limit beyond which the cannula 22 cannot be inserted into the trachea. The holes 224a and 224b allow, through the use of conventional fastening straps (not shown), to fix the cannula 22 and keep it in the correct position during the movements of the head and swallowing.

During use, the fluid connection between the small tube 225 and the cuff 226 allows inflating, through the pilot balloon 227, the cuff 226 of the cannula 22, thus creating a tight coupling between the cuff 226 and the trachea.

The cannula 22 is preferably made of biocompatible plastic material, such as for instance PVC, silicone, etc.

The mandrel 23 has a curved rod 231 ending at an end with a maneuvering knob 232 and, at the opposite end, with an anchoring element 233.

The rod 231 of the mandrel 23 has a curvature similar to that of the body 221 of the cannula 22, so as to be easily inserted thereinto and a similar length, so as to protrude the anchoring element 233 from the first end 222 of the cannula 22.

The knob 232 is useful to maneuver the mandrel 23 when it is placed inside the cannula 22.

The anchoring element 233 of the mandrel 23 is in the shape of a cylindrical body, rounded at its free end (or tip) 234, having a cavity 235 adapted to engage in a removable manner, when in use in association with the cannula 22, with the tooth 214 of the guide tube 212 of the dilator tube 21, thus anchoring the cannula 22 to the guide tube 212. The anchoring system tooth 214/cavity 235 is made so as to ensure an optimal seal of the cannula 22/guide tube 212 assembly until they are aligned with each other and to facilitate the unhooking of the cannula 22 from the guide tube 212 when the cannula 22/mandrel 23 assembly bends with respect to the axis of the tube guide 212.

Advantageously, this hooking/unhooking system allows overcoming the prior art problems relating to the cone-cannula 5 management.

Advantageously, the rounded tip 234 prevents injury to the tracheal lumen and therefore makes it safe to maneuver the mandrel during its insertion into the trachea.

Figure 6 shows in detail the hooking/unhooking system of the cannula 22/mandrel 23 assembly with the guide tube 212 of the kit 20 for extrusive tracheotomy according to alternative variants of the present technology.

In the first variant shown on the left in Figure 6, the guide tube 212 separated from the dilation portion 211 has a flute-beak-like beveled end 213a with the hooking means (tooth 214) arranged therein on the longer side at said beveled end 213a and the cannula 22 has a flute-beak-like beveled end 222a having a complementary profile to that of the beveled end 213a of the guide tube 212.

To perform the hooking, the cannula 22 is arranged substantially in axis with the guide tube 212 with its beveled end 222a juxtaposed to the beveled end 213a of complementary profile of the guide tube 212, so as to allow the engagement of the tooth 214 into the cavity 235 of the anchoring element 233.

To perform the unhooking, the cannula 22 may be tilted with respect to the guide tube 212, keeping away its beveled portion 222a from the beveled portion 213a of the guide tube, so as to cause the unhooking of the anchoring element 233 from the tooth 214.

In the second variant shown on the right in Figure 6, the guide tube 212 separated from the dilation portion 211 has a non-beveled end 213b (i.e. a right angle) in proximity to which the hooking means (tooth 214) and the notch 213c are therein arranged opposite each other and the cannula 22 also has a non-beveled end 222b.

To perform the hooking, the cannula 22 is arranged substantially in axis with the guide tube 212 with its non-beveled end 222b juxtaposed to the non-beveled end 213b of the guide tube 212, so as to allow the engagement of the tooth 214 into the cavity 235 of the anchoring element 233.

To perform the unhooking, the cannula 22 may be tilted with respect to the guide tube 212, so as to cause the unhooking of the anchoring element 233 from the tooth 214, unhooking which is facilitated by the presence of the opposite notch 213c.

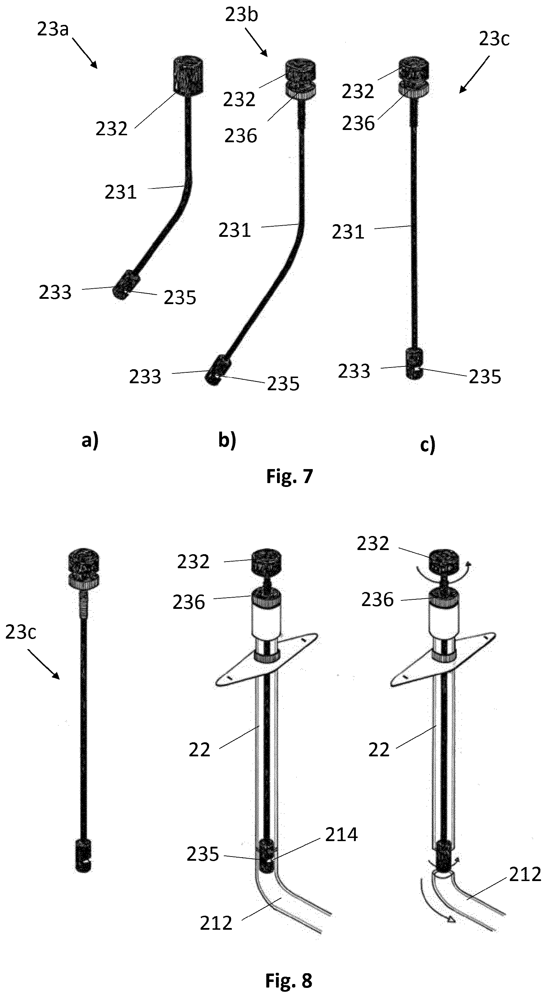

Figure 7 shows several variants of the mandrel 23 of the kit 20 for extrusive tracheotomy subject-matter of the present disclosure.

In detail, the mandrel 23a is the mandrel preferably present in the kit 20 and has been already previously described with reference to Figure 5. The mandrel 23a is fixed and has an ideal shape and length to enter the cannula 22 with flute-beck ends 222a of the kit 20 and to release the anchoring element 233 provided with cavity 235 for the removable engagement with the tooth 214 of the guide tube 212.

Instead, the mandrel 23b is a universal mandrel adapted to adapt with 22 different cannulas if their shape does not allow the use of the mandrel 23a. The mandrel 23b differs from the above-described mandrel 23a substantially in that the rod 231 has a variable length and is therefore provided with a length-adjustment system 236 located at the knob 232.

The adjustment system 236 is preferably of the screw type and allows the mandrel 23b to adapt to different lengths of the cannulas 22 by pushing the cannula 22 to perfectly couple with the guide tube 212, by adjusting the screw of the adjustment system 236 when the mandrel 23b is inserted into the cannula 22 and hooked to the guide tube 212 with right-angled ends 213b.

Advantageously, this allows obtaining a correct coupling between any commercial cannula and the guide tube 212, provided that their outer diameter is substantially the same or similar so as to be compatible.

Advantageously, this allows supplying the kit 20 even without the flute-beak-like cannula 22 but only with the components necessary to perform the extrusive tracheotomy according to the method described in the present disclosure, i.e. the dilator tube 21 and possibly the universal mandrels 23b, 23c and the guide wire 24, using commercial components already in use in the field of intensive medicine for the rest.

Furthermore, the mandrel 23c is a straight universal mandrel, whose rod 231 has no curvature. For this reason, the mandrel 23c is used in cannulas 22 having a particularly soft tubular body 221, which are thus made rectilinear during the insertion step into the trachea.

The cavity 235 of the anchoring element 233 of the mandrel 23c, conversely to the cavities 235 of the mandrels 23a and 23b, allows a hooking and unhooking of the mandrel 23c from the guide tube 212 through a rotation of the mandrel 23c on its main axis by mean of the knob 232.

The knob 232 of the mandrel 23c has an adjustment system 236, preferably a screw-type one, to adapt to different lengths of the cannulas 22.

The mandrels 23a, 23b and 23c are preferably made of a malleable plastic material, for instance PVC, so that their rod 231 is adaptable to the shape and angle of the different cannulas and to the patient's airways.

The mandrels 23a, 23b and 23c may preferably be inserted into the kit at the same time 20.

Advantageously, this allows the doctor to choose the cannula 22 with the most suitable features for the tracheotomy and to always have a type of mandrel 23a, 23b or 23c available to couple it correctly to the guide tube 212.

Advantageously, this allows the kit 20 to integrate with commercial components commonly used in intensive medicine.

Figure 8 shows the mandrel 23c of Figure 7 inserted in the cannula 22 during the hooking step to the guide tube 212 and the unhooking step from the guide tube 212.

The mandrel 23c is inserted in the soft cannula 22 and made straight by the mandrel 23c itself.

The screw adjustment system 236 adjusts the length of the mandrel 23c to ensure that the cavity 235 of the mandrel 23c hooks to the tooth 214 of the guide tube 212, thus ensuring the perfect coupling of the cannula 22 and of the guide tube 212.

In this way, the cannula 22 in use adjacent to the guide tube 212 may be seamlessly inserted into the tracheal stoma (not shown) by pulling the guide tube 212, already inserted in the trachea, towards the patient's mouth.

When the cannula 22 is inserted in the tracheal stoma (not shown), it is possible to unhook the mandrel 23c from the guide tube 212.

The unhooking of the mandrel 23c from the guide tube 212 takes place by rotating the mandrel 23c on its axis through the rotation of the end 232 of the mandrel 23c operated by the doctor.

As for Figure 2, the other components of the extrusive tracheotomy kit 20 present in an embodiment of the present disclosure will now be described.

The guide wire 24 has a "J" soft tip 241, a cable 242 and a knob 243.

The cable 242 is made of braided steel and has two ends.

One end of the cable 242 is joined to the "J" tip 241, whereas the other end of the cable 242 is joined to the knob 243.

The knob 243 is made of plastic material, is cylindrical in shape and suitable for being easily gripped.

The knob 243 is needed to roll up the cable 242 while exerting a controlled traction on the cable 242 during the execution of the tracheotomy.

Advantageously, the cable 242 of the guide wire 24 is preferably already firmly connected to the knob 243 thus eliminating the technical difficulties present in the prior art.

The ventilation small tube 25 is per se conventional and has an elongated and thin tubular shaped body 251 and two ends 252 and 253 and a small tube 255.

The end 252 of the ventilation small tube 25 is at right angle and has, likewise to the end 222 of the cannula 22, an inflatable cuff 254 for the positive pressure ventilation and airway protection.

The end 253 of the ventilation small tube 25 is provided, likewise to the end 223 of the cannula 22, with a universal fitting for the mechanical ventilation.

The small tube 255, likewise to the small tube 225 of the cannula 22, allows inflating, through the pilot balloon 256, the cuff 254 of the ventilation small tube 25, creating a tight coupling between the cuff 254 and the trachea.

It may be appropriate for the ventilation small tube 25 to be long enough to allow cuff 254 to be positioned just above the keel of the trachea, distally with respect to the area where the stoma will be created.

The ventilation small tube 25 preferably has a diameter suitable to allow the simultaneous entry into the trachea of the dilator tube 21 during the execution of the tracheotomy. For instance, in the case of an adult male, in which the cannula 22 could have an internal diameter of 8 mm, the ventilation small tube 25 could have an internal diameter of 5 mm.

The needle 26 must be of adequate caliber to allow the guide wire 24 to enter. Preferably, the needle 26 could be a curved sharp needle 26a or of the "Tuohy" type 26b.

The tracheoscope 27 has an elongated and thin tubular body 271 and two ends 272 and 273 and a small tube 275.

The end 272 of the tracheoscope 27 is flute-beak-like and has, likewise to what has already been described regarding the cannula 22 and the ventilation small tube 25, an inflatable cuff 274 for positive pressure ventilation and airway protection.

The end 273 of the tracheoscope 27 is provided, likewise to what has already been described regarding the cannula 22 and the ventilation small tube 25, with a universal fitting for the mechanical ventilation.

The small tube 275, likewise to what has already been described regarding the cannula 22 and the ventilation small tube 25, allows inflating, through the pilot balloon 276, the cuff 274 of the tracheoscope 27, thus creating a tight coupling between the cuff 274 and the trachea.

The tracheoscope 27 allows access to rigid optics and stabilization of the trachea during the tracheal puncture.

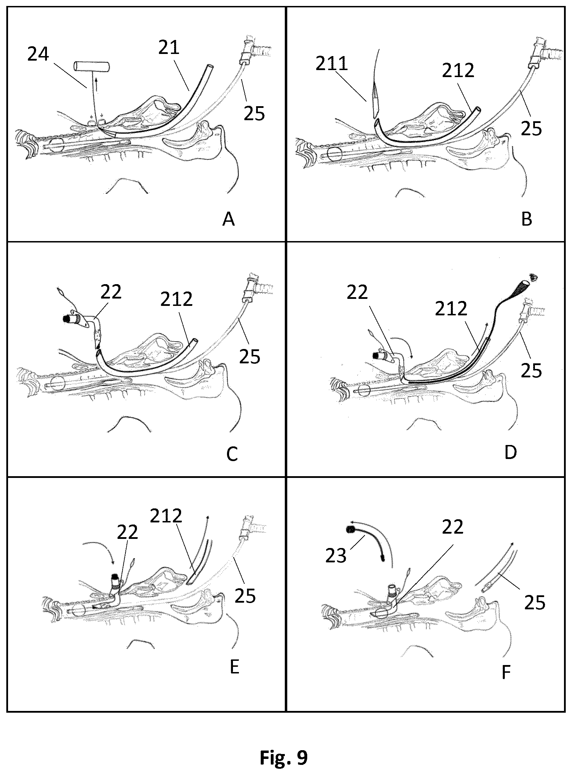

With reference to Figures 1 and 9, a new method for performing extrusive percutaneous dilational tracheotomy with the kit 20 according to the technology will now be described.

The steps of the method are:
1. insertion of a rigid tracheoscope to allow the use of rigid optics and to stabilize and protect the trachea (Fig. 1B) or alternatively use of a fibrobronchoscope and the tube already present in the patient's airways (not shown);
2. puncture of the trachea with the needle 26 under direct tracheoscopic vision (Fig. 1C);
3. insertion of the guide wire 24 into the needle 26 (Fig. 1D);
4. extraction of the guide wire 24 from the mouth, cutting of the "J" tip 241 and reintubation with ventilation small tube 25 (Fig. 1E);
5. insertion of the guide wire 24 into the tip 215 of the dilation portion 211 of the dilator tube 21 and quick hooking thereof to the locking system 217;
6. creation of the stoma by means of the dilation portion 211 by advancing the dilator tube 21 from the inside to the outside by maneuvering the knob 243 of the guide wire 24 (Fig. 9A);
7. separation of the dilation portion 211 from the guide tube 212 by following the flute-beck-like 213a incision line 213 as illustrated in Fig. 9B or following the perpendicular incision line 213b;
8. hooking of the cannula 22/mandrel 23 assembly to the guide tube 212 of the dilator tube 21 (Fig. 9C);
9. insertion of a fibrobronchoscope into the guide tube 212 to observe and control the tracheotomy procedure, gradual progression of the cannula 22/guide tube 212 assembly through the tracheal stoma by pushing the cannula 22 and pulling the guide tube 212 from the mouth, directing the cannula 22 towards the keel of the trachea (Fig. 9D);
10. unhooking of the mandrel 23 from the guide tube 212 through a technique suitable for the type of mandrel, i.e. flexion of the cannula 22 for the mandrel 23a-23b or rotation of the mandrel for the mandrel 23c, and extraction of the fibrobronchoscope and of the guide tube 212 from the mouth (Fig. 9E);
11. extraction of the mandrel 23 from the cannula 22, extraction of the ventilation small tube 25, inflation of the cuff 226 of the cannula 22 and definitive ventilation through the new cannula 22 (Fig. 9F).

In light of the above, the kit of components according to the technology achieves the pre-fixed aims and numerous advantages with respect to the prior art.

Indeed, the kit 20 for extrusive tracheotomy according to the technology advantageously allows performing an extrusive percutaneous dilational tracheotomy in a simpler and safer manner than the prior art techniques and with maneuvers similar to those of the intrusive tracheotomy techniques currently most used in the field of intensive medicine.

Advantageously, this makes the extrusive percutaneous dilational tracheotomy technique faster and easier to learn and perform.

In particular, the advantages of this extrusive tracheotomy technique with respect to the TLT extrusive tracheotomy technique are:
- shorter duration of execution;
- easy management of the guide wire 24: due to its connection with the knob 243 and its quick and firm connection to the sole tip 215 of the dilation portion 211 of the dilator tube 21 through the hooking system 217, complications in the management of the guide wire 24 are avoided and the risk of cutting the guide wire 24 is eliminated during the separation of the guide tube 212 from the dilation portion 211;
- easy cutting of the dilation portion 211: being it connected to the guide tube 212 and not to the cannula 22, there is no risk of cutting the small tube 225 of the cuff necessary to inflate the cuff 226 of the cannula 22;
- elimination of the cannula 22 reorientation maneuver;
- possibility of using any preferred commercial cannula 22 whose diameter is compatible with the diameter of the guide tube 212.

In light of what has been described above, the kit 20 for extrusive percutaneous dilational tracheotomy of the present technology allows performing an extrusive tracheotomy, thus reducing the drawbacks of the prior art.

Yet, the kit 20 for extrusive percutaneous dilational tracheotomy of the present disclosure may contain components that are universal and usable with the commercial ones normally used in the field of intensive medicine.

Finally, the kit 20 for extrusive percutaneous dilational tracheotomy of the present disclosure is easy and simple to make.

A skilled person may make numerous changes and variants to the kit of components according to the invention, by the way all included in the scope of protection of the attached claims.

## Claims

1. Kit (20) of components for extrusive percutaneous dilational tracheotomy comprising a dilator tube (21) that is extended along a longitudinal axis and having a dilation tapered portion (211) ending with a tip (215) and a guide tubular portion (212), **characterized in that** said dilation tapered portion (211) and said guide tubular portion (212) are separatable from each other along at least one weakening line (211b; 212a), preferably an incision line, which is transversely extended with respect to said longitudinal axis, the guide tubular portion (212) further comprising, in proximity to said weakening line (211b; 212a), hooking means (214) for a mandrel (23a; 23b; 23c) associated with a cannula (22).

2. Kit (20) of components according to claim 1, wherein the tapered portion (211) of the dilator tube (21) has a channel (216) therein, which is longitudinally extended from the tip (215) and unidirectional locking means (217) for a guide wire (24) in said channel (216).

3. Kit (20) of components according to claim 1 or 2, wherein the at least one weakening line (211b; 212a) between the dilation tapered portion (211) and the guide tubular portion (212) of said dilator tube (21) is transversely obliquely extended so as to form, after separation, a guide tube (212) with a beveled end (213a) having a substantially flute-beak-like cross portion and having said hooking means (214) in proximity to said beveled end (213a), the kit further comprising a cannula (22) having a flute-beak-like beveled distal end (222) having a profile that is complementary to that of the beveled end (213a) of said guide tube (212).

4. Kit (20) of components according to claim 1 or 2, wherein the at least one weakening line (211b; 212a) between the dilation tapered portion (211) and the guide tubular portion (212) of said dilator tube (21) is perpendicularly extended with respect to the longitudinal axis, so as to form, after separation, a guide tube (212) with a non-beveled end (213b) and having said hooking means (214) in proximity to said non-beveled end (213b), the guide tube (212) further having a longitudinal notch (213c) in proximity to said weakening line (211b; 212a), said longitudinal notch (213c) being substantially opposite said hooking means (214).

5. Kit (20) of components according to claim 3 or 4, comprising a weakening line (212a) that is transversely obliquely extended with respect to the longitudinal axis of the dilator tube (21) and a weakening line (211b) that is perpendicularly extended with respect to the longitudinal axis of the dilator tube (21).

6. Kit (20) of components according to any one of the previous claims, further comprising a cannula (22) having a curved tubular body (221) ending with a flute-beak-like beveled or non-beveled distal end (222) and an opposite proximal end (223), a flange (224) in proximity to the proximal end (223) and a cuff (226) in proximity to the distal end (222) in fluid communication with a balloon (227) through a small tube (225), the cannula preferably further having a safety opening that is known as a "Murphy eye" in proximity to the distal end (222) and/or further comprising a mandrel (23a; 23b; 23c) that is associable with a corresponding cannula (22), the mandrel (23a; 23b; 23c) comprising a rod (231) with a proximal end and a distal end, a manoeuvring knob (232) at the proximal end and an anchoring element (233) provided with a cavity (235) at the opposite distal end, the anchoring element (233) being adapted to removably engage with the hooking means (214) of the guide tube (212).

7. Kit (20) of components according to claim 6, wherein the anchoring element (233) of the mandrel (23) has a substantially cylindrical body that is rounded at the free end thereof (234).

8. Kit (20) of components according to claim 6 or 7, wherein the rod (231) of the mandrel is curved and has a fixed length with a curvature and length substantially corresponding to those of a curved cannula (22) which it is intended to be associated with.

9. Kit (20) of components according to claim 6 or 7, wherein the rod (231) of the mandrel is curved or rectilinear with a variable length and the mandrel comprises an adjustment system (236) for adjusting the length of the rod (231).

10. Kit (20) of components according to any one of the previous claims, further comprising at least one additional component selected from a guide wire (24), a ventilation small tube (25), at least one needle (26) and a tracheoscope (27).

## Patentansprüche

1. Komponenten-Kit (20) für die extrusive perkutane Dilatationstracheotomie, das ein Dilatatorrohr (21) aufweist, das sich entlang einer Längsachse erstreckt und einen sich verjüngenden Dilatationsteil (211), der in einer Spitze (215) endet, und einen rohrförmigen Führungsteil (212) hat, **dadurch gekennzeichnet, dass** der sich verjüngende Dilatationsteil (211) und der rohrförmige Führungsteil (212) entlang mindestens einer Schwächungslinie (211b; 212a), vorzugsweise einer Schnittlinie, voneinander trennbar sind, die sich quer zu der Längsachse erstreckt, wobei der rohrförmige Führungsteil (212) ferner Einhakmittel (214) in der Nähe zu der Schwächungslinie (211b; 212a) für einen mit einer Kanüle (22) verbundenen Dorn (23a; 23b; 23c) aufweist.

2. Komponenten-Kit (20) nach Anspruch 1, bei dem der sich verjüngende Teil (211) des Dilatatorrohres (21) einen Kanal (216) darin hat, der sich von der Spitze (215) aus in Längsrichtung erstreckt, und unidirektionale Verriegelungsmittel (217) für einen Führungsdraht (24) in dem Kanal (216) hat.

3. Komponenten-Kit (20) nach Anspruch 1 oder 2, bei dem sich die mindestens eine Schwächungslinie (211b; 212a) zwischen dem sich verjüngenden Dilatationsteil (211) und dem rohrförmigen Führungsteil (212) des Dilatatorrohres (21) in Querrichtung schräg erstreckt, um nach der Trennung ein Führungsrohr (212) mit einem abgeschrägten Ende (213a) zu bilden, das einen im Wesentlichen flötenschnabelähnlichen Querschnitt hat und bei dem die Einhakmittel (214) in der Nähe des abgeschrägten Endes (213a) sind, wobei das Kit ferner eine Kanüle (22) aufweist, die ein flötenschnabelähnliches abgeschrägtes distales Ende (222) mit einem Profil hat, das komplementär zu dem des abgeschrägten Endes (213a) des Führungsrohres (212) ist.

4. Komponenten-Kit (20) nach Anspruch 1 oder 2, bei dem sich die mindestens eine Schwächungslinie (211b; 212a) zwischen dem sich verjüngenden Dilatationsteil (211) und dem rohrförmigen Führungsteil (212) des Dilatatorrohres (21) senkrecht zu der Längsachse erstreckt, um nach der Trennung ein Führungsrohr (212) mit einem nicht abgeschrägten Ende (213b) zu bilden und bei dem die Einhakmittel (214) in der Nähe des nicht abgeschrägten Endes (213b) sind, wobei das Führungsrohr (212) ferner eine longitudinale Kerbe (213c) in der Nähe der Schwächungslinie (211b; 212a) hat, wobei die longitudinale Kerbe (213c) im Wesentlichen gegenüber der Einhakmittel (214) ist.

5. Komponenten-Kit (20) nach Anspruch 3 oder 4, mit einer Schwächungslinie (212a), die sich in Querrichtung schräg zu der Längsachse des Diatatorrohres (21) erstreckt, und einer Schwächungslinie (211b), die sich senkrecht zu der Längsachse des Dilatatorrohres (21) erstreckt.

6. Komponenten-Kit (20) nach einem der vorhergehenden Ansprüche, ferner mit einer Kanüle (22), die einen gebogenen rohrförmigen Körper (221) hat, der in einem flötenschnabelähnlichen abgeschrägten oder nicht abgeschrägten distalen Ende (222) und einem gegenüberliegenden proximalen Ende (223) endet, einem Flansch (224) in der Nähe des proximalen Endes (223) und einem Cuff (226) in der Nähe des distalen Endes (222) in Fluidverbindung mit einem Ballon (227) über ein kleines Rohr (225), wobei die Kanüle vorzugsweise ferner eine als "Murphy-Auge" bekannte Sicherheitsöffnung in der Nähe des distalen Endes (222) hat und/oder ferner einen Dorn (23a; 23b; 23c) aufweist, der mit einer entsprechenden Kanüle (22) verbindbar ist, wobei der Dorn (23a; 23b; 23c) einen Stab (231) mit einem proximalen Ende und einem distalen Ende, einen Manövrierknopf (232) an dem proximalen Ende und ein Verankerungselement (233) aufweist, das mit einem Hohlraum (235) an dem gegenüberliegenden distalen Ende versehen ist, wobei das Verankerungselement (233) ausgebildet ist, um mit den Hakenmitteln (214) des Führungsrohres (212) lösbar in Eingriff zu kommen.

7. Komponenten-Kit (20) nach Anspruch 6, bei dem das Verankerungselement (233) des Dornes (23) einen im Wesentlichen zylindrischen Körper hat, der an seinem freien Ende (234) abgerundet ist.

8. Komponenten-Kit (20) nach Anspruch 6 oder 7, bei dem der Stab (231) des Dornes gebogen ist und eine feste Länge mit einer Krümmung und Länge hat, die im Wesentlichen denen einer gebogenen Kanüle (22) entsprechen, die mit ihm zu verbunden ist.

9. Komponenten-Kit (20) nach Anspruch 6 oder 7, bei dem der Stab (231) des Dornes gebogen oder gerade ist und eine variable Länge hat und der Dorn ein Einstellsystem (236) zum Einstellen der Länge des Stabes (231) aufweist.

10. Komponenten-Kit (20) nach irgendeinem der vorhergehenden Ansprüche, ferner mit mindestens einer zusätzlichen Komponente, die zwischen einem Führungsdraht (24), einem kleinen Belüftungsloch (25), mindestens einer Nadel (26) und einem Tracheoskop (27) ausgewählt ist.

## Revendications

1. Kit (20) de composants pour trachéotomie par dilatation percutanée extrusive comprenant un tube dilatateur (21) qui s'étend le long d'un axe longitudinal et présentant une partie conique de dilatation (211) se terminant par une pointe (215) et une partie tubulaire de guidage (212), **caractérisé en ce que** ladite partie conique de dilatation (211) et ladite partie tubulaire de guidage (212) sont séparables l'une de l'autre le long d'au moins une ligne d'affaiblissement (211b ; 212a), de préférence une ligne d'incision, qui s'étend transversalement par rapport audit axe longitudinal, la partie tubulaire de guidage (212) comprenant en outre, à proximité de ladite ligne d'affaiblissement (211b ; 212a), des moyens d'accrochage (214) pour un mandrin (23a ; 23b ; 23c) associé à une canule (22).

2. Kit (20) de composants selon la revendication 1, dans lequel la partie conique (211) du tube dilatateur (21) présente un canal (216) en son sein, qui s'étend longitudinalement à partir de la pointe (215) et des moyens de verrouillage unidirectionnels (217) pour un fil-guide (24) dans ledit canal (216).

3. Kit (20) de composants selon la revendication 1 ou 2, dans lequel l'au moins une ligne d'affaiblissement (211b ; 212a) entre la partie conique de dilatation (211) et la partie tubulaire de guidage (212) dudit tube dilatateur (21) est étendue obliquement transversalement de manière à former, après séparation, un tube de guidage (212) ayant une extrémité biseautée (213a) présentant une partie transversale sensiblement de type bec de cannelure et présentant lesdits moyens d'accrochage (214) à proximité de ladite extrémité biseautée (213a), le kit comprenant en outre une canule (22) présentant une extrémité distale biseautée de type bec de cannelure (222) présentant un profil qui est complémentaire à celui de l'extrémité biseautée (213a) dudit tube de guidage (212).

4. Kit (20) de composants selon la revendication 1 ou 2, dans lequel l'au moins une ligne d'affaiblissement (211b ; 212a) entre la partie conique de dilatation (211) et la partie tubulaire de guidage (212) dudit tube dilatateur (21) est étendue perpendiculairement par rapport à l'axe longitudinal, de manière à former, après séparation, un tube de guidage (212) avec une extrémité non biseautée (213b) et présentant lesdits moyens d'accrochage (214) à proximité de ladite extrémité non biseautée (213b), le tube de guidage (212) présentant en outre une encoche longitudinale (213c) à proximité de ladite ligne d'affaiblissement (211b ; 212a), ladite encoche longitudinale (213c) étant sensiblement opposée auxdits moyens d'accrochage (214).

5. Kit (20) de composants selon la revendication 3 ou 4, comprenant une ligne d'affaiblissement (212a) qui s'étend transversalement obliquement par rapport à l'axe longitudinal du tube dilatateur (21) et une ligne d'affaiblissement (211b) qui s'étend perpendiculairement par rapport à l'axe longitudinal du tube dilatateur (21).

6. Kit (20) de composants selon l'une quelconque des revendications précédentes, comprenant en outre une canule (22) présentant un corps tubulaire incurvé (221) se terminant par une extrémité distale biseautée ou non biseautée de type bec de cannelure (222) et une extrémité proximale opposée (223), une bride (224) à proximité de l'extrémité proximale (223) et un manchon (226) à proximité de l'extrémité distale (222) en communication fluidique avec un ballonnet (227) à travers un petit tube (225), la canule présentant de préférence en outre une ouverture de sécurité connue sous le nom d'« œil de Murphy » à proximité de l'extrémité distale (222) et/ou comprenant en outre un mandrin (23a ; 23b ; 23c) qui peut être associé à une canule (22) correspondante, le mandrin (23a ; 23b ; 23c) comprenant une tige (231) avec une extrémité proximale et une extrémité distale, un bouton de manœuvre (232) au niveau de l'extrémité proximale et un élément d'ancrage (233) doté d'une cavité (235) au niveau de l'extrémité distale opposée, l'élément d'ancrage (233) étant adapté pour entrer en prise de manière amovible avec les moyens d'accrochage (214) du tube de guidage (212).

7. Kit (20) de composants selon la revendication 6, dans lequel l'élément d'ancrage (233) du mandrin (23) présente un corps sensiblement cylindrique qui est arrondi au niveau de son extrémité libre (234).

8. Kit (20) de composants selon la revendication 6 ou 7, dans lequel la tige (231) du mandrin est incurvée et présente une longueur fixe avec une courbure et une longueur correspondant sensiblement à celles d'une canule incurvée (22) à laquelle elle est destinée à être associée.

9. Kit (20) de composants selon la revendication 6 ou 7, dans lequel la tige (231) du mandrin est incurvée ou rectiligne avec une longueur variable et le mandrin comprend un système de réglage (236) pour régler la longueur de la tige (231).

10. Kit (20) de composants selon l'une quelconque des revendications précédentes, comprenant en outre au moins un composant supplémentaire choisi parmi un fil-guide (24), un petit tube de ventilation (25), au moins une aiguille (26) et un trachéoscope (27).
